# EUROPÄISCHE PATENTANMELDUNG

(11) **EP 4 299 102 A1**
(43) Veröffentlichungstag der Anmeldung: **03.01.2024**
(21) Anmeldenummer: 22181301.7
(22) Anmeldetag: 27.06.2022
(51) Int. Cl.: A61M 60/178, A61M 60/216, A61M 60/585, A61M 60/508, A61M 60/871, H01M 50/262

(54) **SICHERHEITSMECHANISMUS, SICHERHEITSSYSTEM, VAD-SYSTEM SOWIE VERFAHREN ZUM LÖSEN ZWEIER EINHEITEN, GEKOPPELT DURCH EIN ENTSPRECHENDES SICHERHEITSSYSTEM**

(71) Anmelder: Berlin Heart GmbH, 12247 Berlin (DE)
(72) Erfinder: SCHÄRFF, Eike, 12167 Berlin (DE)
(74) Vertreter: Pfenning, Meinig & Partner mbB

(57) **Zusammenfassung**

Die vorliegende Offenbarung betrifft einen Sicherheitsmechanismus zum Verriegeln und Lösen einer ersten mit einer zweiten Einheit, insbesondere zum Verriegeln und Lösen einer Akkueinheit (1) eines VAD-Systems mit einer Kontrolleinheit (2) eines VAD-Systems. Der Sicherheitsmechanismus umfasst einen Rasthaken (21) aufweisend eine Rastnase (22), eine Rastöffnung (11) und ein erstes Betätigungselement (12). In einer Ruheposition greift der Rasthaken (21) in die Rastöffnung (11) derart ein, dass der Rasthaken (21) durch die Rastöffnung (11) hindurchragt und die Rastnase (22) eine Umrandung der Rastöffnung (11) zumindest teilweise hintergreift. In einer Vorlöseposition greift der Rasthaken (21) zumindest teilweise in die Rastöffnung (11) derart ein, dass zumindest ein Teil des Rasthakens (21) durch die Rastöffnung hindurchragt und die Rastnase (22) von der Umrandung der Rastöffnung (11) gelöst ist. In einer Freigabeposition ist der Rasthaken (21) außerhalb der Rastöffnung (11) angeordnet ist. Dabei ist das Betätigungselement derart ausgebildet, dass eine Betätigung des Betätigungselements (12) den Rasthaken (21) und/oder die Rastöffnung (11) von der Vorlöseposition in die Freigabeposition bewegt.

## Beschreibung

Die vorliegende Offenbarung betrifft einen Sicherheitsmechanismus zum Verriegeln und Lösen einer ersten mit einer zweiten Einheit, insbesondere zum Verriegeln und Lösen einer Akkueinheit eines VAD-Systems mit einer Kontrolleinheit eines VAD-Systems. Ein VAD-System ist ein Herzunterstützungssystem, wobei VAD für Ventricular Assist Device steht. Ferner betrifft die vorliegende Offenbarung ein Sicherheitssystem und ein VAD-System, jeweils umfassend einen entsprechenden Sicherheitsmechanismus. Die vorliegende Offenbarung betrifft zudem ein Verfahren zum Lösen eines entsprechenden Sicherheitsmechanismus.

Im Stand der Technik sind Herzunterstützungssysteme bekannt. Ein ventrikuläres Unterstützungssystem (VAD) ist ein elektromechanisches Gerät zur Unterstützung des Herzkreislaufs, das entweder teilweise oder vollständig die Funktion eines versagenden Herzens ersetzen soll. VAD-Systeme umfassen typischerweise eine implantierbare Herzpumpe, eine Steuereinheit, die die Herzpumpe steuert sowie eine Batterie- oder Akkueinheit zur Versorgung der Steuereinheit und/oder der Herzpumpe mit Strom. Die Kontrolleinheit und die Akku- oder Batterieeinheit sind dabei typischerweise außerhalb eines Patientenkörpers angeordnet. Um eine einwandfreie Funktion des VAD-Systems gewährleisten zu können, muss eine sichere Stromversorgung gewährleistet werden. Die Akkueinheit oder Batterieeinheit muss daher sicher an der Kontrolleinheit befestigt sein. Um Batterien oder Akkus zu wechseln, muss die Akku- oder Batterieeinheit von der Kontrolleinheit lösbar sein. Um die Stromversorgung der Herzpumpe und/oder der Kontrolleinheit nicht zu gefährden, muss gewährleistet werden, dass die Akku- oder Batterieeinheit und die Kontrolleinheit nicht versehentlich voneinander getrennt werden.

Die vorliegende Erfindung hat daher die Aufgabe, eine sichere und lösbare Verbindung zwischen einer Akku- oder Batterieeinheit und einer Kontrolleinheit, insbesondere für ein VAD-System, vorzuschlagen.

Diese Aufgabe wird gelöst durch einen Sicherheitsmechanismus gemäß Anspruch 1. Ferner wird diese Aufgabe gelöst durch ein Sicherheitssystem und/oder ein VAD-System und/oder ein Verfahren gemäß den nebengeordneten Ansprüchen. Vorteilhafte Weiterbildungen der Erfindung sind in den Unteransprüchen dargelegt.

Die dieser Offenbarung beigefügten Figuren sowie die dazugehörige Figurenbeschreibung dienen der Veranschaulichung exemplarischer Ausführungsformen der Erfindung und sind nicht einschränkend zu verstehen. Insbesondere sind die dort beschriebenen Merkmale und Merkmalskombinationen nicht zwingend und lediglich beispielhaft zusammengestellt.

Der vorgeschlagene Sicherheitsmechanismus dient zum Verriegeln und Lösen einer ersten mit einer zweiten Einheit. Insbesondere dient der vorgeschlagene Sicherheitsmechanismus zum lösbaren Verriegeln einer Akkueinheit eines VAD-Systems mit einer Kontrolleinheit eines VAD-Systems.

In der vorliegenden Beschreibung wird der Begriff "Akkueinheit" verwendet, die dem Wortlaut nach einen Akku umfassen kann. Diese Begrifflichkeit ist jedoch nicht einschränkend zu verstehen. So kann die Akkueinheit zusätzlich oder alternativ eine Batterie umfassen.

Der Sicherheitsmechanismus umfasst einen Rasthaken aufweisend eine Rastnase, eine Rastöffnung und ein erstes Betätigungselement. Der Rasthaken kann zwischen einer Ruheposition, einer Vorlöseposition und einer Freigabeposition verbringbar sein. Dafür kann der Rasthaken von einer in die andere Position aktiv durch Bewegung des Rasthakens verbracht werden. Alternativ oder zusätzlich kann ein anderes Bauteil, beispielsweise die Rastöffnung, bewegt werden, sodass der Rasthaken zwischen der Ruheposition, der Vorlöseposition und der Freigabeposition verbracht wird.

In der Ruheposition greift der Rasthaken in die Rastöffnung derart ein, dass der Rasthaken durch die Rastöffnung hindurchragt und die Rastnase eine Umrandung der Rastöffnung zumindest teilweise hintergreift.

In der Vorlöseposition greift der Rasthaken zumindest teilweise in die Rastöffnung derart ein, dass zumindest ein Teil des Rasthakens durch die Rastöffnung hindurchragt und die Rastnase von der Umrandung der Rastöffnung gelöst ist. In der Freigabeposition ist der Rasthaken außerhalb der Rastöffnung angeordnet.

Das Betätigungselement ist derart ausgebildet, dass eine Betätigung des Betätigungselements den Rasthaken und/oder die Rastöffnung von der Vorlöseposition in die Freigabeposition bewegt.

Dies kann den Vorteil haben, dass der Rasthaken von der Rastöffnung nur durch eine zusätzliche Betätigung gelöst werden kann, sodass ein nicht intendiertes Lösen des Rasthakens von der Rastöffnung möglichst vermieden werden kann.

In einer vorteilhaften Ausführungsform können der Rasthaken und die Rastöffnung derart ausgebildet sein, dass in der Ruheposition der Rasthaken mit der Rastöffnung in einer ersten Richtung und in einer zweiten Richtung verriegelt ist und der Rasthaken in eine Richtung zum Verbringen in die Vorlöseposition bewegbar ist. Die zweite Richtung kann insbesondere perpendikulär zur ersten Richtung sein.

Insbesondere kann die zweite Richtung einer Betätigungsrichtung des Betätigungselements entsprechen. Beispielsweise kann ein Eindrücken eines Druccknopfes gesperrt sein. Die Richtung zum Verbringen in die Vorlöseposition kann perpendikulär zur ersten Richtung und/oder zweiten Richtung sein. Insbesondere kann die Richtung zum Verbringen in die Vorlöseposition den Rasthaken entlang seiner Längsachse und/oder die Rastöffnung verschieben. In der Ruheposition kann der Rasthaken beispielsweise nur eine vordefinierte Wegstrecke entlang seiner Längsachse bewegbar sein. Beispielsweise kann der Rasthaken nur in die Vorlöseposition bewegbar sein. Insbesondere kann der Rasthaken aus der Ruhestellung nicht unmittelbar in die Freigabeposition bewegbar sein, also insbesondere ohne dass er vorher in die Vorlöseposition verbracht wurde und ohne dass in der Vorlöseposition das Betätigungselement betätigt wurde.

So kann vorgesehen sein, dass der Rasthaken von einem Nutzer nur durch eine definierte Bewegung, insbesondere durch eine Bewegung der ersten Einheit in Richtung der zweiten Einheit, in die Vorlöseposition verbringbar ist. In der Vorlöseposition kann der Rasthaken derart angeordnet sein, dass er in die Rastöffnung derart eingreift, dass der Rasthaken mit der Rastöffnung in der ersten Richtung verriegelt ist und in der zweiten Richtung gelöst ist. Derart kann vorgesehen sein, dass der Rasthaken entlang der ersten Richtung nicht von der Rastöffnung gelöst werden kann. Jedoch kann in der Vorlöseposition das Betätigungselement derart freigegeben sein, dass dieses betätigbar ist. Das Betätigungselement kann derart ausgebildet sein, dass eine Betätigung gesperrt ist, wenn sich der Rasthaken in der Ruheposition befindet. Die Betätigung des Betätigungselements kann beispielsweise durch die Rastnase in der Ruheposition gesperrt sein. So kann eine versehentliche Betätigung des Betätigungselements möglichst vermieden werden.

Das Betätigungselement kann beispielsweise als Druckelement ausgebildet sein. Insbesondere kann es derart ausgebildet sein, dass eine auf das Betätigungselement ausgeübte Druckkraft den Rasthaken und/oder die Rastöffnung derart bewegt, dass der Rasthaken von der Vorlöseposition in die Freigabeposition verbracht wird. In der Freigabeposition können die Einheit an der der Rasthaken angeordnet ist und die Einheit an der die Rastöffnung angeordnet ist voneinander getrennt werden. Durch die oben beschriebenen Schritte, die ausgeführt werden müssen, um die Einheit an der der Rasthaken angeordnet ist und die Einheit an der die Rastöffnung angeordnet ist voneinander zu trennen, d.h. von der Ruheposition in Vorlöseposition und von dort in die Freigabeposition zu verbringen, kann die Sicherheit des Sicherheitsmechanismus erhöht werden.

In einer bevorzugten Ausführungsform ist das Betätigungselement mit der Rastöffnung fest verbunden. Eine Betätigung des Betätigungselements (die insbesondere nur möglich ist, wenn sich der Rasthaken in der Vorlöseposition befindet) kann die Rastöffnung entlang der zweiten Richtung verschieben. Die Verschiebung der Rastöffnung kann dazu führen, dass der Rasthaken aus der Rastöffnung herausbewegt wird. So kann der Rasthaken nach Betätigung des Betätigungselements in die Freigabeposition verbracht sein. Alternativ kann vorgesehen sein, dass der Rasthaken mit dem Betätigungselement fest verbunden ist. Eine Betätigung des Betätigungselements (die insbesondere nur möglich ist, wenn sich der Rasthaken in der Vorlöseposition befindet) kann den Rasthaken dann derart bewegen, dass dieser aus der Rastöffnung herausbewegt wird.

Der Rasthaken und/oder die Rastöffnung können beispielsweise aus einem Kunststoff sein und/oder Kunststoff umfassen. Der Rasthaken und/oder die Rastöffnung können insbesondere aus einem Material sein, das zumindest teilweise elastisch biegbar ist. So kann eine Betätigung des Betätigungselements beispielsweise den Rasthaken und/oder die Rastöffnung derart elastisch verbiegen, dass der Rasthaken aus der Rastöffnung herausbewegt wird bzw. die Rastöffnung von dem Rasthaken weg bewegt wird. Wird das Betätigungselement nicht mehr betätigt, kann der Rasthaken bzw. die Rastöffnung sich elastisch in die ursprüngliche Position zurückbewegen.

In einer bevorzugten Ausführungsform können der Rasthaken und die Rastöffnung wie nachfolgend beschrieben gelöst werden, wobei als Ausgangsposition der Rasthaken in Ruheposition verriegelt mit der Rastöffnung vorliegt. Wenn der Rasthaken mit der Rastöffnung in Ruheposition in Eingriff ist, sind die erste und die zweite Einheit typischerweise miteinander verriegelt Aus der Ruheposition kann der Rasthaken in die Vorlöseposition verbracht werden. In der Vorlöseposition kann das Betätigungselement betätigt werden. Während das Betätigungselement weiter betätigt wird, kann die erste Einheit von der zweiten Einheit gelöst werden.

Eine Betätigung des Betätigungselements ist in der Ruheposition gesperrt. Eine Betätigung des Betätigungselements kann durch einen Formschluss gesperrt sein. Zum Sperren kann beispielsweise die Rastnase des Rasthakens eine Umrandung der Rastöffnung hintergreifen.

In der Ruheposition können der Rasthaken und die Rastöffnung in die Vorlöseposition bewegbar sein. Die Rastöffnung kann beispielsweise als Langloch ausgebildet sein, in dem der Rasthaken verschiebbar ist. Der Querschnitt eines Teils des Rasthakens, der durch die Rastöffnung ragt, kann dabei also insbesondere kleiner sein als die Rastöffnung. So kann ein Spiel zwischen dem Rasthaken und der Rastöffnung vorgesehen sein. Eine relative Bewegung der ersten Einheit und der zweiten Einheit kann bewirken, dass sich der Rasthaken in der Rastöffnung verschiebt. Die Form und Größe der Rastöffnung kann dabei die Bewegung des Rasthakens in der Rastöffnung begrenzen. Um den Rasthaken in der Rastöffnung zu bewegen, kann beispielsweise die erste Einheit in Richtung der zweiten Einheit bewegt werden und/oder die zweite Einheit kann in Richtung der ersten Einheit bewegt werden. Insbesondere kann diese Bewegung entlang einer Längsachse des Rasthakens verlaufen. In der Ruheposition kann der Rasthaken an einer ersten Position in der als Langloch ausgebildeten Rastöffnung angeordnet sein und in der Vorlöseposition kann der Rasthaken an einer zweiten Position in der als Langloch ausgebildeten Rastöffnung angeordnet sein. Das Langloch kann derart ausgebildet sein, dass der Rasthaken zumindest 1 mm, vorzugsweise zumindest 2 mm, besonders bevorzugt zumindest 3 mm in der Rastöffnung bewegbar ist, wenn sich der Rasthaken in der Ruheposition befindet. Das Langloch kann derart ausgebildet sein, dass der Rasthaken höchstens 6 mm, vorzugsweise höchstens 5 mm, besonders bevorzugt höchstens 4 mm in der Rastöffnung bewegbar ist, wenn sich der Rasthaken in der Ruheposition befindet.

In der Vorlöseposition kann eine Betätigung des Betätigungselements freigegeben sein. Insbesondere können/kann die Rastöffnung und/oder der Rasthaken und/oder die Rastnase derart ausgebildet sein, dass in der Vorlöseposition die Rastnase die Umrandung der Rastöffnung nicht hintergreift. In der Vorlöseposition kann der Rasthaken teilweise durch die Rastöffnung ragen. Die Rastnase kann dabei derart unterhalb oder oberhalb der Rastöffnung aber innerhalb der Umrandung der Rastöffnung liegen, dass eine Betätigung des Betätigungselements, beispielweise ein Eindrücken, freigegeben ist. Die Betätigung des Betätigungselements kann beispielsweise die Rastöffnung derart bewegen, dass die Rastöffnung von dem Rasthaken wegbewegt wird, sodass der Rasthaken in der Freigabeposition ist. Alternativ kann die Betätigung des Betätigungselements beispielsweise den Rasthaken derart bewegen, dass dieser aus der Rastöffnung herausbewegt wird, sodass der Rasthaken in der Freigabeposition ist. An dem Betätigungselement kann ein erstes elastisches Element, beispielsweise eine Feder, angeordnet sein, dass das Betätigungselement in eine Ausgangsposition drängt, in der die Rastöffnung und der Rasthaken miteinander verriegelt sind. Zum Betätigen des Betätigungselements kann also vorgesehen sein, dass eine Kraft auf das Betätigungselement ausgeübt werden muss, die größer ist, als eine elastische Kraft des ersten elastischen Elements, die das Betätigungselement in die Ausgangslage drängt. Ist das erste elastische Element beispielsweise eine Feder, so muss eine Kraft zum Betätigen des Betätigungselements größer als die Federkraft sein. Alternativ oder zusätzlich zu einem ersten elastischen Element kann das Betätigungselement selbst ein elastisches Material umfassen. Die Betätigung des Betätigungselements kann dann beispielsweise die Rastöffnung oder das Element, das die Rastöffnung umfasst, derart verbiegen, dass die Rastöffnung von dem Rasthaken weggebogen wird, sodass der Rasthaken in der Freigabeposition ist. Das Betätigungselement kann dafür insbesondere integral mit der Rastöffnung ausgebildet sein. Alternativ kann die Betätigung des Betätigungselements beispielsweise den Rasthaken oder ein Element, das den Rasthaken umfasst, derart verbiegen, dass der Rasthaken von der Rastöffnung weggebogen wird, sodass der Rasthaken in der Freigabeposition ist. Das Betätigungselement kann dafür insbesondere integral mit dem Rasthaken ausgebildet sein.

In einer bevorzugten Ausführungsform ist vorgesehen, dass - um die erste Einheit von der zweiten Einheit zu lösen - die erste Einheit und die zweite Einheit auseinandergezogen werden, wenn sich der Rasthaken in der Freigabeposition befindet. Insbesondere kann vorgesehen sein, dass die erste Einheit und die zweite Einheit, bzw. der Rasthaken und die Rastöffnung, entlang einer Richtung entlang der oder parallel zur Längsachse des Rasthakens auseinandergezogen werden müssen. Dabei kann insbesondere vorgesehen sein, dass das Betätigungselement weiterhin betätigt bleiben muss, damit der Rasthaken in der Freigabeposition verbleibt.

In einer bevorzugten Ausführungsform umfasst der Sicherheitsmechanismus ein zweites elastisches Element, das derart ausgebildet und angeordnet ist, dass es den Rasthaken von der Vorlöseposition in die Ruheposition drängt. Das zweite elastische Element kann beispielsweise als Feder ausgebildet sein oder eine Feder umfassen. Alternativ oder zusätzlich kann das elastische Element ein Elastomer, beispielsweise in Form einer Gummidichtung, umfassen oder bilden. Eine Gummidichtung kann an der ersten und/oder der zweiten Einheit partiell angeordnet oder umlaufend ausgebildet sein. Insbesondere kann die Gummidichtung umlaufend an einer Kontaktfläche zwischen der ersten und der zweiten Einheit angeordnet sein. Die Gummidichtung kann die erste Einheit mit der zweiten Einheit abdichten. Eine Gummidichtung kann den Vorteil haben, dass die neben ihrer elastischen Eigenschaft einen Gehäuseinnenraum, der in der ersten und /oder zweiten Einheit angeordnet ist, bspw. wasser- und/oder gasdicht und/oder staubdicht abdichten kann, solange die erste und die zweite Einheit verbunden sind. Die Gummidichtung kann auch im Wesentlichen keine oder wenig dichtenden Eigenschaften aufweisen und lediglich der elastischen Verbindung zwischen erster und zweiter Einheit dienen.

In einer Ausführung umfasst der Sicherheitsmechanismus einen zweiten Rasthaken, eine zweite Rastöffnung und ein zweites Betätigungselement. Der zweite Rasthaken kann spiegelsymmetrisch zum ersten Rasthaken ausgebildet und/oder angeordnet sein. Die zweite Rastöffnung kann spiegelsymmetrisch zur zweiten Rastöffnung ausgebildet und/oder angeordnet sein. Das zweite Betätigungselement kann spiegelsymmetrisch zum ersten Betätigungselement ausgebildet und/oder angeordnet sein.

Die oben beschriebenen Merkmale bezüglich des ersten Rasthakens und/oder der ersten Rastöffnung und/oder des ersten Betätigungselement können auch auf den zweiten Rasthaken und/oder die zweite Rastöffnung und/oder das zweite Betätigungselement angewandt werden.

In einer bevorzugten Ausbildung ist der oben beschriebene Rasthaken an einer ersten Seite der ersten Einheit angeordnet und dazu spiegelsymmetrisch an einer zweiten Seite der ersten Einheit ein gleich ausgebildeter Rasthaken. In dieser Ausführungsform ist an der zweiten Einheit die oben beschriebene Rastöffnung an einer ersten Seite der ersten Einheit angeordnet und dazu spiegelsymmetrisch an einer zweiten Seite der zweiten Einheit eine gleich ausgebildeter Rastöffnung. Dies hat den Vorteil, dass ein Lösen der ersten Einheit von der zweiten Einheit eine Betätigung, insbesondere eine gleichzeitige Betätigung, des ersten und des zweiten Betätigungselements erfordert. Dies kann den Sicherheitsmechanismus sicherer machen, da ein nicht intendiertes Lösen unwahrscheinlicher ist.

Das erste und das zweite Betätigungselement können gegenüber angeordnet sein. Insbesondere können das erste und das zweite Betätigungselement derart gegenüber angeordnet sein, dass ein gleichzeitiges Zusammendrücken der Betätigungselemente bewirkt, dass sich der erste und der zweite Rasthaken in der Freigabeposition befinden. Insbesondere können das erste und das zweite Betätigungselement derart gegenüber angeordnet sein, dass ein Abstand zwischen den Betätigungselementen maximal 7 cm, vorzugsweise maximal 6 cm, besonders bevorzugt maximal 4 cm beträgt. Insbesondere können das erste und das zweite Betätigungselement derart gegenüber angeordnet sein, dass ein Abstand zwischen den Betätigungselementen minimal 1 cm, vorzugsweise minimal 2 cm, besonders bevorzugt minimal 3 cm beträgt. Insbesondere können das erste und das zweite Betätigungselement derart gegenüber angeordnet sein, dass ein erwachsener Nutzer das erste und das zweite Betätigungselement mit einer Hand, d.h. mit der rechten Hand oder der linken Hand, betätigen kann, beispielsweise mit einem Daumen und einem Zeigefinger. Insbesondere kann ein Abstand so gewählt sein, dass die Handgröße vom 5. Perzentil bis zum 95. Perzentil der einzusetzenden Benutzer ausreicht, um das erste und das zweite Betätigungselement gleichzeitig zu bedienen, insbesondere gemäß DIN 33402-2:2005-12.

Insbesondere können das erste und das zweite Betätigungselement derart gegenüber angeordnet sein, dass ein Kind von 6 Jahren das erste und das zweite Betätigungselement mit einer Hand betätigen kann, beispielsweise mit einem Daumen und einem Zeigefinger. Insbesondere kann ein maximaler Abstand größer als 2 cm, vorzugsweise größer als 3 cm, besonders bevorzugt größer als 3,5 cm sein. Insbesondere kann ein maximaler Abstand kleiner als 8 cm, vorzugsweise kleiner als 6 cm, besonders bevorzugt kleiner als 5 cm sein. Insbesondere können das erste und das zweite Betätigungselement derart gegenüber angeordnet sein, dass ein Kind von 3 Jahren oder jünger das erste und das zweite Betätigungselement mit einer Hand, beispielsweise mit einem Daumen und einem Zeigefinger, nicht betätigen kann.

Um ein versehentliches Lösen der ersten Einheit von der zweiten Einheit zu vermeiden, kann ein weiteres Sicherheitselement vorgesehen sein. Das Sicherheitselement kann von einer Sicherheitsposition in eine Löseposition verbringbar sein. In der Sicherheitsposition kann das Sicherheitselement eine Betätigung des Betätigungselements sperren. In der Löseposition kann das Sicherheitselement eine Betätigung des Betätigungselements freigeben. In diesem Ausführungsbeispiel müsste zum Lösen der ersten Einheit von der zweiten Einheit zunächst (wie auch in den obigen Ausführungsbeispielen) der Rasthaken von der Ruheposition in die Vorlöseposition verbracht werden. In der Vorlöseposition müsste daraufhin das Sicherheitselement von der Sicherheitsposition in die Löseposition verbracht werden, damit das Betätigungselement betätigt werden kann. Sollten ein erstes und ein zweites Betätigungselement vorgesehen sein, so kann das Sicherheitselement in der Sicherheitsposition gleichzeitig das erste und das zweite Betätigungselement sperren. Ein Verbringen des Sicherheitselements in die Löseposition kann das erste und das zweite Betätigungselement gleichzeitig freigeben. Alternativ kann ein erstes Sicherheitselement vorgesehen sein, dass in der Sicherheitsposition das erste Betätigungselement sperrt und ein zweites Sicherheitselement vorgesehen sein, das in der Sicherheitsposition das zweite Betätigungselement sperrt. Zum Betätigen des ersten und zweiten Betätigungselements müssten dann sowohl das erste als auch das zweite Sicherheitselement von der Sicherheitsposition in die Löseposition verbracht werden. In der Löseposition kann daraufhin das (erste und/oder zweite) Betätigungselement betätigt werden, um die erste Einheit von der zweiten Einheit lösen zu können.

Die vorliegende Offenbarung betrifft ferner ein Sicherheitssystem umfassend die erste Einheit und die zweite Einheit, wobei die erste Einheit mit der zweiten Einheit über den oben beschriebenen Sicherheitsmechanismus lösbar verbindbar ist. Insbesondere ist dabei der Rasthaken fest mit der ersten Einheit verbunden und/oder Teil der ersten Einheit. Die Rastöffnung kann fest mit der zweiten Einheit verbunden sein und/oder Teil der zweiten Einheit sein. Der Rasthaken kann mit der ersten Einheit integral ausgebildet sein und/oder mit der ersten Einheit verschweißt, verklammert und/oder verschraubt sein. Die Rastöffnung kann mit der zweiten Einheit integral ausgebildet sein und/oder mit der zweiten Einheit verschweißt, verklammert und/oder verschraubt sein.

Die erste Einheit und die zweite Einheit weisen insbesondere eine elektrische Verbindung auf, die trennbar ist. Insbesondere ist die elektrische Verbindung gegeben, wenn die erste und die zweite Einheit miteinander verriegelt sind. Insbesondere ist die elektrische Verbindung gegeben, wenn die erste und die zweite Einheit, bzw. der Rasthaken und die Rastöffnung, in der Ruhestellung und/oder in der Vorlöseposition sind. Die elektrische Verbindung kann einen Stecker und eine Buchse aufweisen. Insbesondere kann der Stecker an der ersten Einheit angeordnet sein und die Buchse an der zweiten Einheit angeordnet sein oder umgekehrt. Wenn die erste und die zweite Einheit eine Akkueinheit und eine Kontrolleinheit sind und diese miteinander verriegelt sind, wird die Steuereinheit insbesondere von dem Akku bzw. der Batterie der Ackueinheit mit Strom versorgt.

In einer vorteilhaften Ausbildung ist die erste Einheit ein Kunststoff-Spritzgussteil und der Rasthaken ist einteilig mit der ersten Einheit ausgebildet. In einer vorteilhaften Ausbildung ist die zweite Einheit ein Kunststoff-Spritzgussteil und die Rastöffnung ist einteilig mit der ersten Einheit ausgebildet. Beispielsweise kann die erste Einheit und/oder die zweite Einheit eines oder mehrere der nachfolgenden Materialien umfassen oder aus einem dieser hergestellt sein: Polypropylen (PP) und/oder Acrylnitrit-Butadien-Styrol (ABS) und/oder Polycarbonat (PC) und/oder Polymethylmethacrylat (PMMA) und/oder Aliphatische Polyamide (PPA) und/oder Polyoxymethylen (POM) und/oder Polybutylenterephthalat (PBT) und/oder Polyetherimid (PEI) und/oder Polyethylen (PE).

Insbesondere sind die erste Einheit und die zweite Einheit in der Ruheposition und in der Vorlöseposition über den Sicherheitsmechanismus miteinander verriegelt und in der Freigabeposition voneinander gelöst.

Die vorliegende Offenbarung betrifft ferner ein VAD-System umfassend ein oben beschriebenes Sicherheitssystem. Das VAD-System kann ferner eine Herzpumpe umfassen. Die erste Einheit kann eine Kontrolleinheit sein. Die zweite Einheit kann eine Batterieeinheit oder eine Akkueinheit sein. Alternativ kann die erste Einheit eine Batterieeinheit oder eine Akkueinheit sein und die zweite Einheit eine Kontrolleinheit sein.

Ferner betrifft die vorliegende Offenbarung ein Verfahren zum Lösen der ersten Einheit von der zweiten Einheit des oben beschriebenen Systems, insbesondere des oben beschriebenen VAD-Systems.

Das Verfahren umfasst die Schritte
I. "Bewegen der ersten Einheit in Richtung der zweiten Einheit"
II. Betätigen des Betätigungselements
III. Lösen der ersten Einheit von der zweiten Einheit,
wobei Schritt II auf Schritt I folgt und Schritt III auf Schritt II folgt, um die erste Einheit von der zweiten Einheit zu lösen.

In einer Ausführungsvariante des Verfahrens kann zwischen den Schritten I und II ein Sicherheitselement von einer Sicherheitsposition in eine Löseposition verbracht werden. Das Sicherheitselement kann in der Sicherheitsposition eine Betätigung des Betätigungselements sperren und in der Löseposition eine Betätigung des Betätigungselements freigeben.

In den Figuren werden beispielhafte Ausführungen der Erfindung näher erläutert. Es zeigen
- Fig. 1a: eine Kontrolleinheit und eine Akkueinheit eines VAD-Systems in per-spektivischer Ansicht, wobei die Kontrolleinheit und die Akkueinheit miteinander verriegelt sind,
- Fig. 1b: die Kontrolleinheit und die Akkueinheit der Fig. 1a in perspektivischer Ansicht, wobei die Kontrolleinheit und die Akkueinheit voneinander gelöst sind,
- Fig. 1c: die Kontrolleinheit und die Akkueinheit der Fign. 1a und 1b in einer Draufsicht in einer Ruheposition,
- Fig. 1d: die Kontrolleinheit und die Akkueinheit der Fig. 1c in einer Schnittansicht entlang des in Figur 1c eingezeichneten Schnittes AA in der Ruheposition,
- Fig. 1e: die Kontrolleinheit und die Akkueinheit der Fign. 1a bis 1d in einer Draufsicht in einer Vorlöseposition,
- Fig. 1f: die Kontrolleinheit und die Akkueinheit der Fig. 1a-1e in einer Schnittansicht entlang des in Figur 1e eingezeichneten Schnittes AA in der Vorlöseposition,
- Fig. 1g: eine Detailansicht des Betätigungselements und des Rasthakens der vorherigen Figuren in der Freigabeposition,
- Fig. 1h: die Kontrolleinheit und die Akkueinheit der Fig. 1a-1g in einer Schnittansicht entlang des in Figur 1e eingezeichneten Schnittes AA in der Freigabeposition,
- Fig. 1i: ein VAD-System in Ruheposition, das entsprechend dem VAD-System der Figuren 1a bis 1f ausgebildet ist, wobei anstelle einer Feder ein Elastomer als elastisches Element zwischen der ersten und der zweiten Einheit vorgesehen ist,
- Fig. 1j: ein VAD-System in Vorlöseposition, das entsprechend dem VAD-Sys-tem der Figuren 1a bis 1f ausgebildet ist, wobei anstelle einer Feder ein Elastomer als elastisches Element zwischen der ersten und der zweiten Einheit vorgesehen ist,
- Fig. 2a: ein VAD-System in perspektivischer Ansicht, wobei die Kontrolleinheit und die Akkueinheit voneinander gelöst sind,
- Fig. 2b: die Kontrolleinheit und die Akkueinheit der Fig. 2a in einer Draufsicht in einer Ruheposition, in der die Kontrolleinheit und die Akkueinheit miteinander verriegelt sind
- Fig. 2c: die Kontrolleinheit und die Akkueinheit der Fig. 2b in einer Schnittansicht entlang des in Figur 2b eingezeichneten Schnittes BB in der Ruheposition,
- Fig. 2d: die Kontrolleinheit und die Akkueinheit der Fign. 2b und 2c in einer Schnittansicht entlang des in Figur 2c eingezeichneten Schnittes AA in der Ruheposition,
- Fig. 2e: die Kontrolleinheit und die Akkueinheit der Fign. 2a bis 2d in einer Draufsicht in einer Vorlöseposition,
- Fig. 2f: die Kontrolleinheit und die Akkueinheit der Fig. 2e in einer Schnittansicht entlang des in Figur 2e eingezeichneten Schnittes BB in der Vorlöseposition,
- Fig. 2g: die Kontrolleinheit und die Akkueinheit der Fign. 2e und 2f in einer Schnittansicht entlang des in Figur 2f eingezeichneten Schnittes AA in der Vorlöseposition,
- Fig. 2h-j: ein VAD-System, das entsprechend des VAD-Systems der Figuren 2a bis 2g ausgebildet ist, wobei anstelle einer Feder ein Elastomer als elastisches Element zwischen der ersten und der zweiten Einheit vorgesehen ist, wobei 2h einen Schnitt AA, Fig. 2i einen Schnitt BB und Fig. 2j eine Draufsicht zeigt,
- Fig. 3a: ein VAD-System in perspektivischer Ansicht, wobei die Kontrolleinheit und die Akkueinheit voneinander gelöst sind,
- Fig. 3b: die Kontrolleinheit und die Akkueinheit der Fig. 3a in einer Draufsicht in einer Ruheposition, in der die Kontrolleinheit und die Akkueinheit miteinander verriegelt sind,
- Fig. 3c: die Kontrolleinheit und die Akkueinheit der Fig. 3b in einer Schnittansicht entlang des in Figur 3b eingezeichneten Schnittes AA in der Ruheposition,
- Fig. 3d: die Kontrolleinheit und die Akkueinheit der Fign. 2b und 2c in einer Schnittansicht entlang des in Figur 3c eingezeichneten Schnittes BB in der Ruheposition,
- Fig. 3e: die Kontrolleinheit und die Akkueinheit der Fign. 3a bis 3d in einer Draufsicht in einer Freigabeposition,
- Fig. 3f: die Kontrolleinheit und die Akkueinheit der Fig. 3e in einer Schnittansicht entlang des in Figur 3e eingezeichneten Schnittes AA in der Freigabeposition,
- Fig. 3g: die Kontrolleinheit und die Akkueinheit der Fig. 3f in einer Schnittansicht entlang des in Figur 3f eingezeichneten Schnittes BB in der Freigabeposition,
- Fig. 3h-j: ein VAD-System, das entsprechend des VAD-Systems der Figuren 3a bis 3g ausgebildet ist, wobei anstelle einer Feder ein Elastomer als elastisches Element zwischen der ersten und der zweiten Einheit vorgesehen ist, wobei 3h einen Schnitt BB, Fig. 3i einen Schnitt AA und Fig. 2j eine Draufsicht zeigt.

Wiederkehrende Merkmale werden in den Figuren mit denselben Bezugszeichen versehen.

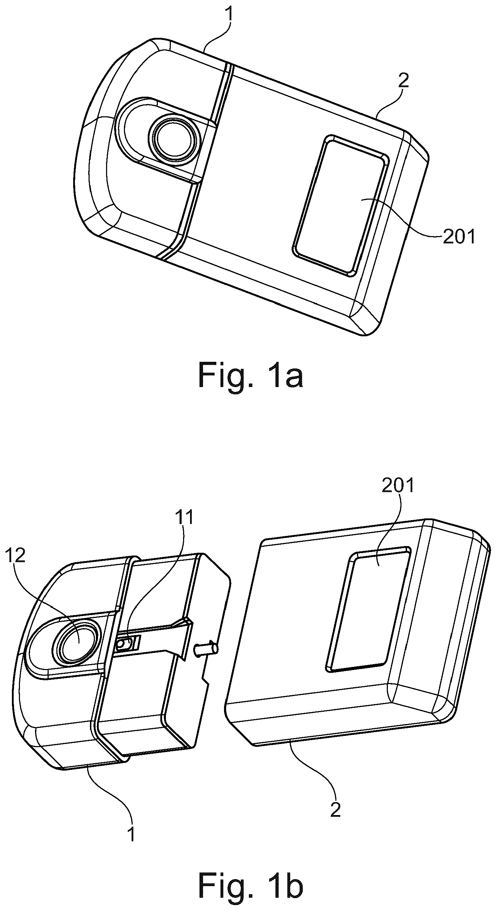

In Figur 1a ist eine Kontrolleinheit 2 und eine Akkueinheit 1 eines VAD-Systems in einer perspektivischen Ansicht gezeigt. Die Akkueinheit 1 umfasst einen Akku zum Versorgen der Kontrolleinheit 2 mit Strom. Die Kontrolleinheit 2 umfasst ein elektronisches Gerät, beispielsweise eine Steuereinheit. Die Steuereinheit ist eingerichtet zum Steuern einer Herzpumpe. Die Herzpumpe (nicht gezeigt) ist mit der Steuereinheit kabellos oder über Kabel verbunden. Die Kontrolleinheit 2 umfasst ein Display 201 zum Anzeigen verschiedener Informationen, beispielsweise zum Anzeigen einer Akkuladung des Akkus der Akkueinheit 1 und/oder zum Anzeigen von Herzdaten und/oder zum Anzeigen von Daten der Herzpumpe.

Die Akkueinheit 1 und die Kontrolleinheit 2 sind über einen Sicherheitsmechanismus lösbar miteinander verriegelt. Figur 1a zeigt das VAD-System in einer Ruheposition. Fig. 1b zeigt die Akkueinheit 1 und die Kontrolleinheit 2 in einer voneinander gelösten Position. Der Sicherheitsmechanismus zum lösbaren Verriegeln der Akkueinheit 1 mit der Kontrolleinheit 2 umfasst einen Rasthaken 21, eine Rastöffnung 11 und ein erstes Betätigungselement 12. Das Betätigungselement 12 sowie die Rastöffnung 11 sind Teil der Akkueinheit 1. Der Rasthaken 21 ist Teil der Kontrolleinheit 2.

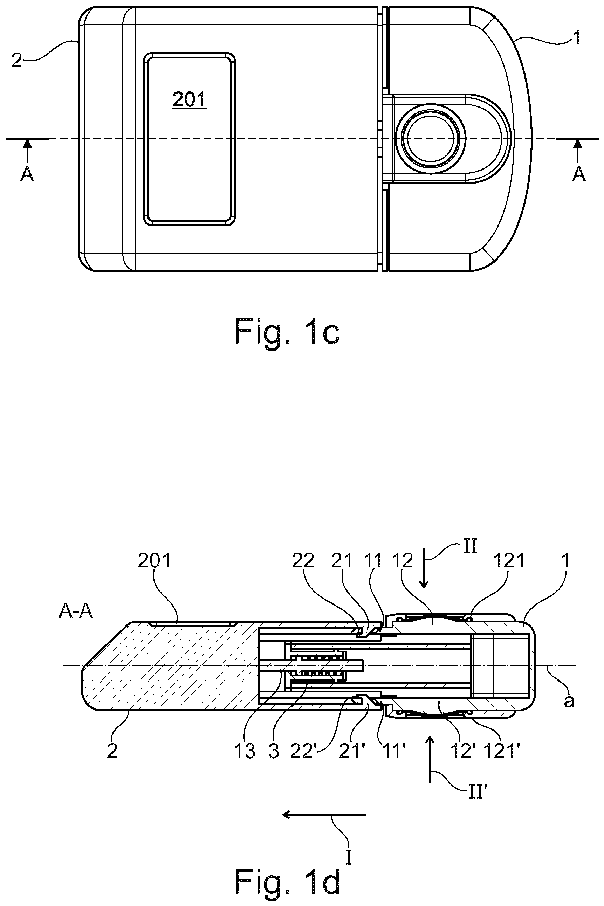

Figur 1c zeigt die Akkueinheit 1 und die Kontrolleinheit 2 in einer verriegelten Ruheposition in einer Draufsicht. Figur 1d zeigt die Akkueinheit 1 und die Kontrolleinheit 2 in einer verriegelten Ruheposition in einer Schnittansicht entlang des in Figur 1c eingezeichneten Schnitts AA. Der Rasthaken 21 ist integral mit dem Gehäuse der Kontrolleinheit 2 ausgebildet. Der Rasthaken weist eine Rastnase 22 auf. Das Betätigungselement 12 ist integral mit dem Gehäuse der Akkueinheit 1 und integral mit der Rastöffnung 11 ausgebildet. In der in Fig. 1c und Fig. 1d dargestellten Ruheposition ragt der Rasthaken 21 durch die Rastöffnung 11 hindurch. Die Rastnase 22 hintergreift eine Umrandung der Rastöffnung 11, sodass eine Betätigung des Betätigungselements 12 in einer Richtung II gesperrt ist.

Spiegelsymmetrisch zu einer Achse a weist die Akkueinheit 1 ein zweites Betätigungselement 12' und eine zweite Rastöffnung 11' auf, die integral mit dem Gehäuse der Akkueinheit 1 ausgebildet sind. Ebenfalls spiegelsymmetrisch zur Achse a weist die Kontrolleinheit 2 einen zweiten Rasthaken 21' mit einer zweiten Rastnase 22' auf. Der Rasthaken 21' ist integral mit dem Gehäuse der Kontrolleinheit 2 ausgebildet. In der in Figur 1d dargestellten Ruheposition ragt der Rasthaken 21' durch die Rastöffnung 11' hindurch. Die Rastnase 22' hintergreift eine Umrandung der Rastöffnung 11', sodass eine Betätigung des Betätigungselements 12' in einer Richtung II' gesperrt ist.

Eine Feder 3 ist in der Akkueinheit 1 angeordnet. In der Ruheposition drängt die Feder 3 die Kontrolleinheit 2 entlang der Achse a weg von der Akkueinheit 1. Im vorliegend gezeigten Ausführungsbeispiel ist die Feder 3 eine Spiralfeder. Die Akkueinheit umfasst einen Stift 13, der translatorisch beweglich mit dem Gehäuse der Akkueinheit 1 verbunden ist. Der Stift 13 wird von der Feder 3 entlang der Achse a in Richtung I gedrängt. Der Stift 13 kann eine elektrische Verbindung zwischen dem in der Akkueinheit 1 angeordneten Akku bzw. der Batterie und der Kontrolleinheit 2 umfassen. Alternativ kann ein Stecker und eine Buchse anderer Form für die elektrische Verbindung zwischen dem Akku bzw. der Batterie der Akkueinheit 1 und der Kontrolleinheit 2 vorgesehen sein. Der Stecker und die Buchse können derart ausgebildet sein, dass das elastische Element diese auseinanderdrängt. Wenn die Akkueinheit 1 und die Kontrolleinheit zusammengesetzt sind, stößt der Stift 13 an die Kontrolleinheit 2 an. Zum Zusammensetzen der Akkueinheit 1 und der Kontrolleinheit 2 werden die Akkueinheit 1 und Kontrolleinheit 2 zusammengedrückt, wobei eine Druckkraft größer als die Federkraft der Feder 3 ist. In der Ruheposition, d.h. im in Fign. 1c und 1d gezeigten Zustand, übt die Feder 3 eine Kraft auf die Kontrolleinheit 2 aus. Die Feder 3 ist also in der Ruheposition vorgespannt. Wenn keine externe Druckkraft auf die Akkueinheit 1 und/oder die Kontrolleinheit 2 ausgeübt wird, drängt die Feder 3 die Akkueinheit 1 und die Kontrolleinheit entlang der Achse a auseinander. Dabei wird der Rasthaken in die in Figur 1d gezeigte Position in der als Langloch ausgebildeten Rastöffnung 11 gedrängt. In dieser Ruheposition hintergreift die Rastnase 22 die Umrandung der Rastöffnung 11 und die Rastnase 22' die Umrandung der Rastöffnung 11'.

Das Betätigungselement 12 weist eine elastische Membran 121 auf, die auf einer Betätigungsfläche des Betätigungselements12 angeordnet ist. Die elastische Membran 121 dient als Dichtung und verbessert zudem eine Haptik des Betätigungselements 12. Das Betätigungselement 12' weist eine elastische Membran 121' auf, die auf einer Betätigungsfläche des Betätigungselements 12' angeordnet ist. Die elastische Membran 121' dient als Dichtung und verbessert zudem eine Haptik des Betätigungselements 12'. Ein Innenraum der Akkueinheit kann wasser-, gas- und/oder staubdicht abgedichtet sein.

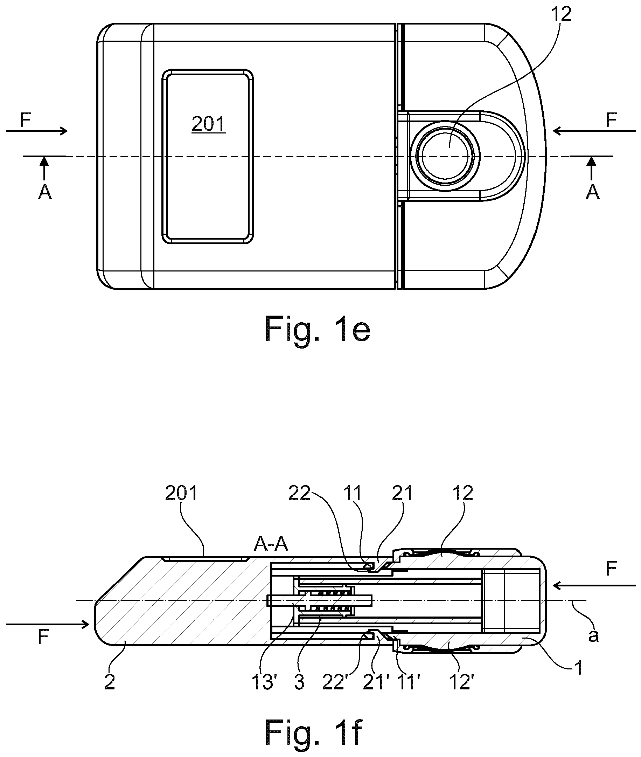

In Figur 1e ist die gleiche Ansicht wie in Figur 1c gezeigt und in Figur 1f ist die gleiche Schnittansicht wie in Figur 1d gezeigt. In den Figuren 1e und 1f sind die Kontrolleinheit 1 und die Akkueinheit 1 jedoch in einer Vorlöseposition gezeigt. Ausgehend von der in Figuren 1c und 1d gezeigten Ruheposition wurden die Akkueinheit 1 in Richtung der Kontrolleinheit 2 dafür entlang der Achse a um 3 mm bewegt. In der Vorlöseposition ragt der Rasthaken 21 weiterhin durch die Rastöffnung 11 und der Rasthaken 21' ragt weiterhin durch die Rastöffnung 11'. Die Rastnase 22 hintergreift jedoch nicht mehr die Umrandung der Rastöffnung 11 und die Rastnase 22' hintergreift nicht mehr die Umrandung der Rastöffnung 11'. Gegenüber der in Figur 1d gezeigten Ruheposition ist die Kontrolleinheit in Figur 1f 3 mm entgegen der Richtung I entlang der Achse a auf die Akkueinheit 1 zu bewegt. Dafür wirkt auf die Kontrolleinheit 2 eine Kraft F, die größer ist als die Federkraft der Feder 3, die die Akkueinheit 1 und die Kontrolleinheit 2 auseinanderdrängt.

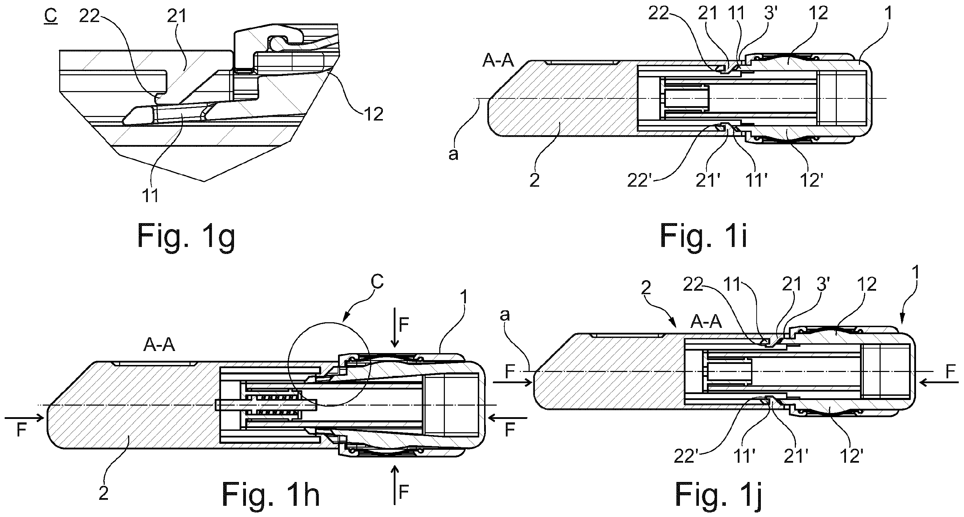

Fig. 1h zeigt die Kontrolleinheit 2 und die Akkueinheit 1 der vorherigen Figuren im Schnitt AA in einer Freigabeposition. In Fig. 1h ist ein Ausschnitt C markiert, der in Fig. 1g in vergrößerter Form dargestellt ist. Fig. 1g zeigt demnach in schematischer Darstellung das Betätigungselement 12, die Rastöffnung 11 und den Rasthaken 22 der der Figuren 1a - 1h. In den Figuren 1h und 1g sind das Betätigungselement 12, die Rastöffnung 11 und der Rasthaken 22 in der Freigabeposition dargestellt. Dabei wirkt auf das Betätigungselement 12 eine Kraft F, die das Betätigungselement 12 zusammen mit der Rastöffnung 11 nach unten auslenkt. Dabei wird der Rasthaken 21 freigegeben. Wenn gleichzeitig auch das Betätigungselement 12' betätigt wird, kann so die Akkueinheit 1 von der Kontrolleinheit 2 gelöst werden. Damit das Betätigungselement 12 und 12' betätigt werden können, müssen die Akkueinheit 1 und die Kontrolleinheit 2 in die Vorlöseposition verbracht sein (wie in Figur 1e und 1f gezeigt), da sonst die Rastnase 22 bzw. 22' eine Auslenkung des Betätigungselements 12 bzw. 12' sperrt. Das Betätigungselement 12 und die Rastöffnung 11 sowie das Betätigungselement 12' und die Rastöffnung 11' sind aus Kunststoff, insbesondere aus PC und/oder ABS und/oder Polyphthalamid (PPA). Die in Fig. 1g bzw. 1h gezeigte Verformung des Betätigungselements 12 (sowie die Verformung des Betätigungselements 12') in die Freigabeposition sind elastisch, sodass sich das Betätigungselement 12 (bzw. das Betätigungselement 12') in die in Figur 1f gezeigte Position zurückbewegt, sobald keine Kraft F mehr auf das Betätigungselement 12 (bzw. 12') ausgeübt wird.

Die Figuren 1i und 1j zeigen in Schnittansicht AA durch eine Kontrolleinheit 2 und eine Akkueinheit 1, die denen der Figuren 1a-g entspricht, wobei anstelle einer Feder 3 ein elastisches Element 3' in Form eines umlaufenden elastischen Dichtelements, beispielsweise ein Moosgummi-Ring, zwischen der Kontrolleinheit 2 und der Akkueinheit 1 angeordnet ist. Die oben in Bezug auf die vorherigen Figuren beschriebenen Merkmale treffen daher auch auf das in Figuren 1i und 1j gezeigte VAD-System zu. Dabei zeigt Fig. 1i die Kontrolleinheit 2 und die Akkueinheit 1 in einem zusammengesetzten und verriegelten Zustand in der Ruheposition. Figur 1j zeigt die Kontrolleinheit 2 und die Akkueinheit 1 der Vorlöseposition. In der Vorlöseposition ist das umlaufende elastische Dichtelement zusammengedrückt durch eine Kraft F, die auf die Akkueinheit wirkt. Die Rastnase 22 hintergreift die Umrandung der Rastöffnung 11 in Fig. 1j nicht.

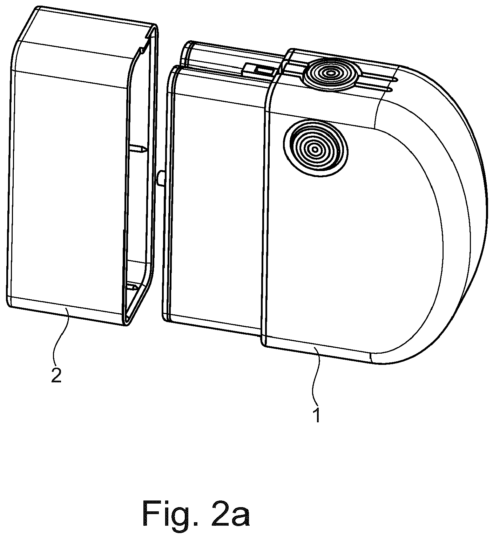

Die Figuren 2a-j zeigen ein VAD-System mit einer alternativen Ausführung eines Sicherheitsmechanismus zum lösbaren Verriegeln einer Akkueinheit 1 mit einer Kontrolleinheit 2. In Figur 2a ist das VAD-System in perspektivischer Ansicht dargestellt, wobei die Akkueinheit 1 und die Kontrolleinheit 2 voneinander gelöst sind.

Die Akkueinheit 1 umfasst einen Akku zum Versorgen der Kontrolleinheit 2 mit Strom. Die Kontrolleinheit 2 umfasst ein elektronisches Gerät, beispielsweise eine Steuereinheit. Die Steuereinheit ist eingerichtet zum Steuern einer Herzpumpe. Die Herzpumpe (nicht gezeigt) ist mit der Steuereinheit kabellos oder über Kabel verbunden. Die Kontrolleinheit 2 kann ein Display (nicht gezeigt) zum Anzeigen verschiedener Informationen, beispielsweise zum Anzeigen einer Akkuladung des Akkus der Akkueinheit 1 und/oder zum Anzeigen von Herzdaten und/oder zum Anzeigen von Daten der Herzpumpe, umfassen.

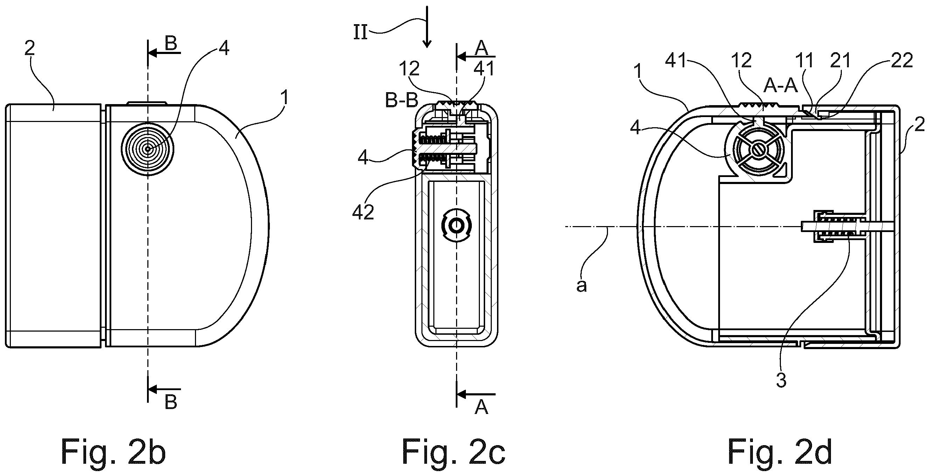

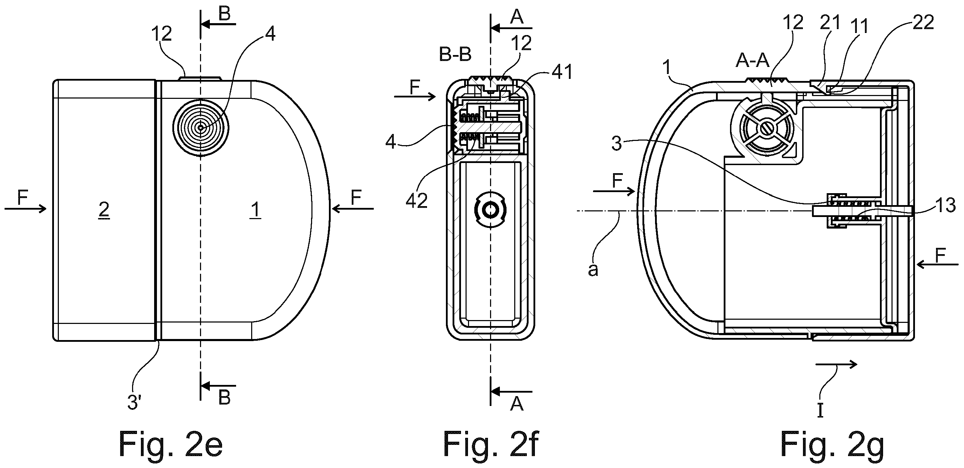

Die Akkueinheit 1 und die Kontrolleinheit 2 sind über einen Sicherheitsmechanismus lösbar miteinander verriegelt. Figuren 2b bis 2d zeigen das VAD-System in einer Ruheposition. Figuren 2e bis 2g zeigen das VAD-System in einer Vorlöseposition. Der Sicherheitsmechanismus zum lösbaren Verriegeln der Akkueinheit 1 mit der Kontrolleinheit 2 umfasst einen Rasthaken 21, eine Rastöffnung 11 und ein Betätigungselement 12. Das Betätigungselement 12 sowie die Rastöffnung 11 sind Teil der Akkueinheit 1. Der Rasthaken 21 ist Teil der Kontrolleinheit 2.

Figur 2b zeigt die Akkueinheit 1 und die Kontrolleinheit 2 in einer verriegelten Ruheposition in einer Draufsicht. In Figur 2c ist ein Schnitt durch die Akkueinheit 1 entlang des in Figur 2b gezeigten Schnitts BB gezeigt. Figur 2d zeigt die Akkueinheit 1 und die Kontrolleinheit 2 in einer verriegelten Ruheposition in einer Schnittansicht entlang des in Figur 2c eingezeichneten Schnitts AA. Figuren 2e bis 2g zeigen die Akkueinheit 1 und die Kontrolleinheit 2 in einer Vorlöseposition in einer Draufsicht. In Figur 2f ist ein Schnitt durch die Akkueinheit 1 entlang des in Figur 2e gezeigten Schnitts BB gezeigt. Figur 2g zeigt die Akkueinheit 1 und die Kontrolleinheit 2 in einer Vorlöseposition in einer Schnittansicht entlang des in Figur 2f eingezeichneten Schnitts AA.

Der Rasthaken 21 ist integral mit dem Gehäuse der Kontrolleinheit 2 ausgebildet. Der Rasthaken weist eine Rastnase 22 auf. Das Betätigungselement 12 ist integral mit dem Gehäuse der Akkueinheit 1 und integral mit der Rastöffnung 11 ausgebildet. In der in den Figuren 2b bis 2d dargestellten Ruheposition ragt der Rasthaken 21 durch die Rastöffnung 11 hindurch. Die Rastnase 22 hintergreift eine Umrandung der Rastöffnung 11, sodass eine Betätigung des Betätigungselements 12 in einer Richtung II gesperrt ist.

Die Akkueinheit 1 weist ein Sicherheitselement 4 auf. Das Sicherheitselement 4 ist von einer Sicherheitsposition in eine Löseposition verbringbar. Das Sicherheitselement 4 sperrt in der Sicherheitsposition eine Betätigung des Betätigungselements 12 und gibt in der Löseposition eine Betätigung des Betätigungselements 12 frei. Das Sicherheitselement 4 ist als Druckknopf ausgebildet. In den Figuren 2b bis 2d ist das Sicherheitselement 4 in der Sicherheitsposition gezeigt. Ein Zapfen 41 des Sicherheitselements 4 sperrt die Betätigung des Betätigungselements 12. Das Sicherheitselement weist eine Feder 42 auf, die das Sicherheitselement in die Sicherheitsposition drängt. Zum Verbringen des Sicherheitselements in die Löseposition muss ein Nutzer eine Kraft F auf das Sicherheitselement 4 aufbringen, die die Feder 42 zusammendrückt (wie in Fig. 2f gezeigt). In den Figuren 2e bis 2g ist das Sicherheitselement 4 in der Löseposition gezeigt. Ein Nutzer bringt eine Kraft F auf das Sicherheitselement auf, sodass der Zapfen 41 des Sicherheitselements 4 eine Betätigung des Betätigungselements 12 freigibt.

Eine Feder 3 ist in der Akkueinheit 1 angeordnet. In der Ruheposition drängt die Feder 3 die Kontrolleinheit 2 entlang der Achse a weg von der Akkueinheit 1. Im vorliegend gezeigten Ausführungsbeispiel ist die Feder 3 eine Spiralfeder. Die Akkueinheit umfasst einen Stift 13, der translatorisch beweglich mit dem Gehäuse der Akkueinheit 1 verbunden ist. Der Stift 13 wird von der Feder 3 entlang der Achse a in Richtung I gedrängt. Wenn die Akkueinheit 1 und die Kontrolleinheit zusammengesetzt sind, stößt der Stift 13 an die Kontrolleinheit 2 an. Zum Zusammensetzen der Akkueinheit 1 und der Kontrolleinheit 2 werden die Akkueinheit 1 und Kontrolleinheit 2 zusammengedrückt, wobei eine Druckkraft größer als die Federkraft der Feder 3 ist. In der Ruheposition, d.h. im in Fign. 2b-2d und 1d gezeigten Zustand, übt die Feder 3 eine Kraft auf die Kontrolleinheit 2 aus. Die Feder 3 ist also in der Ruheposition vorgespannt. Wenn keine externe Kraft auf die Akkueinheit 1 oder die Kontrolleinheit 2 ausgeübt wird, drängt die Feder 3 die Akkueinheit 1 und die Kontrolleinheit 2 entlang der Achse a auseinander. Dabei wird der Rasthaken 21 in die in Figur 2d gezeigte Position in der als Langloch ausgebildeten Rastöffnung 11 gedrängt. In dieser Ruheposition hintergreift die Rastnase 22 die Umrandung der Rastöffnung 11.

Ausgehend von der in Figuren 2b bis 2d gezeigten Ruheposition wurden die Akkueinheit 1 und die Kontrolleinheit 2 entlang der Achse a zusammengedrückt, um die Akkueinheit 1 bzw. die Kontrolleinheit 2 in die in Figuren 2e bis 2g gezeigte Vorlöseposition zu verbringen. In der Vorlöseposition ragt der Rasthaken 21 weiterhin durch die Rastöffnung 11. Die Rastnase 22 hintergreift jedoch nicht mehr die Umrandung der Rastöffnung 11. Gegenüber der in Figur 2d gezeigten Ruheposition ist die Kontrolleinheit in Figur 2e 3 mm entgegen der Richtung I entlang der Achse a auf die Akkueinheit 1 zu bewegt. Dafür wirkt auf die Kontrolleinheit 2 eine Kraft F, die größer ist als die Federkraft der Feder 3, die die Akkueinheit 1 und die Kontrolleinheit 2 auseinanderdrängt.

Zum Verbringen der Akkueinheit 1 und Kontrolleinheit in die Freigabeposition, müssen die Akkueinheit 1 und die Kontrolleinheit 2 also zunächst zusammengedrückt werden, sodass sie sich in der Vorlöseposition befinden. Daraufhin wird das Sicherheitselement 4 betätigt und betätigt gehalten, sodass die Betätigung des Betätigungselements 12 freigegeben ist. Dann wird das Betätigungselement 12 betätigt, wodurch die Rastöffnung nach unten gebogen wird. Nun befindet sich der Rasthaken 21 außerhalb der Rastöffnung 11 (vgl. Fig. 1g). Nun können die Akkueinheit 1 und die Kontrolleinheit 2 voneinander gelöst werden. Die Verformung des Betätigungselements 12 in die Freigabeposition sind elastisch, sodass sich das Betätigungselement 12 in die in Figuren 2e-g gezeigte Position zurückbewegt, sobald keine Kraft F mehr auf das Betätigungselement 12 ausgeübt wird. Wird keine Kraft mehr auf das Sicherheitselement ausgeübt, so wird dieses ebenfalls wieder von einer Feder 42 in ihre Ausgangslage zurückbewegt, in der sie eine Betätigung des Betätigungselements 12 sperrt.

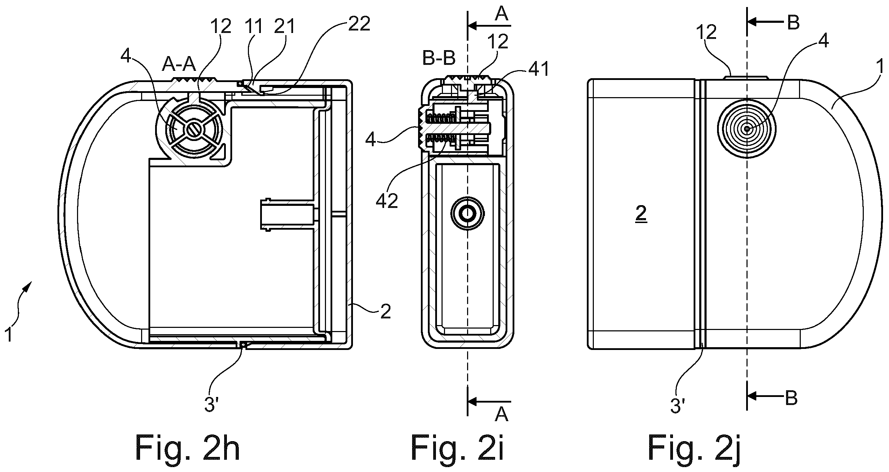

Die Figur 2h zeigt in Schnittansicht AA, die Figur 2i in Schnittansicht BB und Figur 2j in Draufsicht eine Kontrolleinheit 2 und eine Akkueinheit 1, die denen der Figuren 2a-g entspricht, wobei anstelle einer Feder 3 ein elastisches Element 3' in Form eines umlaufenden elastischen Dichtelements, beispielsweise ein Moosgummi-Ring, zwischen der Kontrolleinheit 2 und der Akkueinheit 1 angeordnet ist. Die oben in Bezug auf die vorherigen Figuren beschriebenen Merkmale treffen daher auch auf das in Figuren 2h-j gezeigte VAD-System zu. Dabei zeigen Fig. 2h-j die Kontrolleinheit 2 und die Akkueinheit 1 in einem zusammengesetzten und verriegelten Zustand in der Ruheposition.

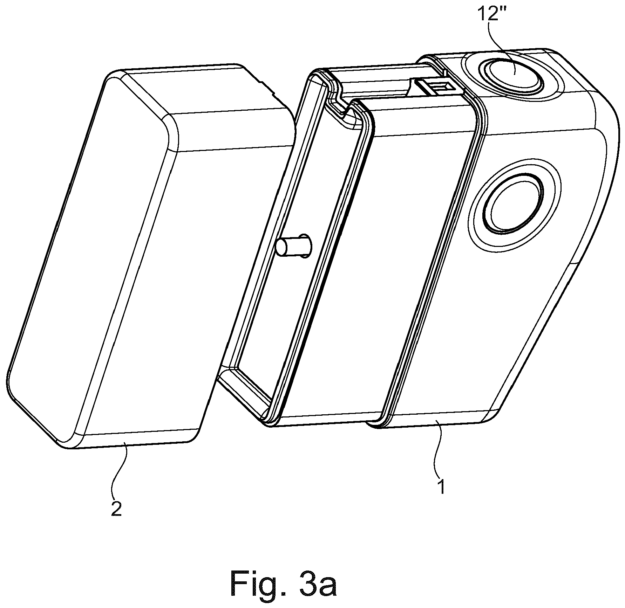

In einer alternativen Ausführung kann das Betätigungselement 12 nicht integral mit dem Gehäuse der Akkueinheit 1 ausgebildet sein, sondern als separates Teil, beispielsweise als Druckknopf 12". Eine derartige Ausführung des Betätigungselementes 12 kann sowohl in dem Ausführungsbeispiel der Figuren 1 als auch in dem Ausführungsbeispiel der Figuren 2 umgesetzt sein. Beispielhaft wird eine derartige Ausführung in den Figuren 3a-j gezeigt. Die Figuren 3a bis 3j zeigen dabei eine Ausführung mit einem Sicherheitselement 4', das ähnlich dem Sicherheitselement 4 der Figuren 2 ist.

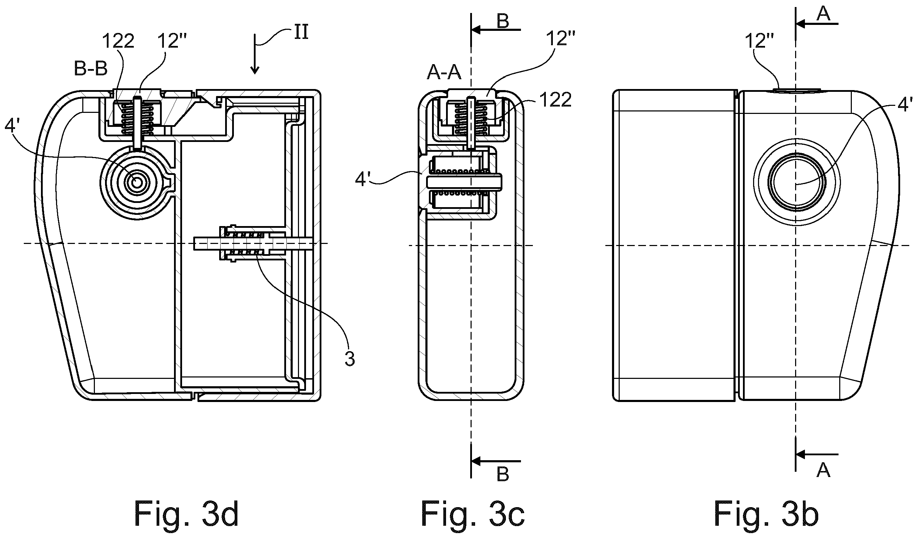

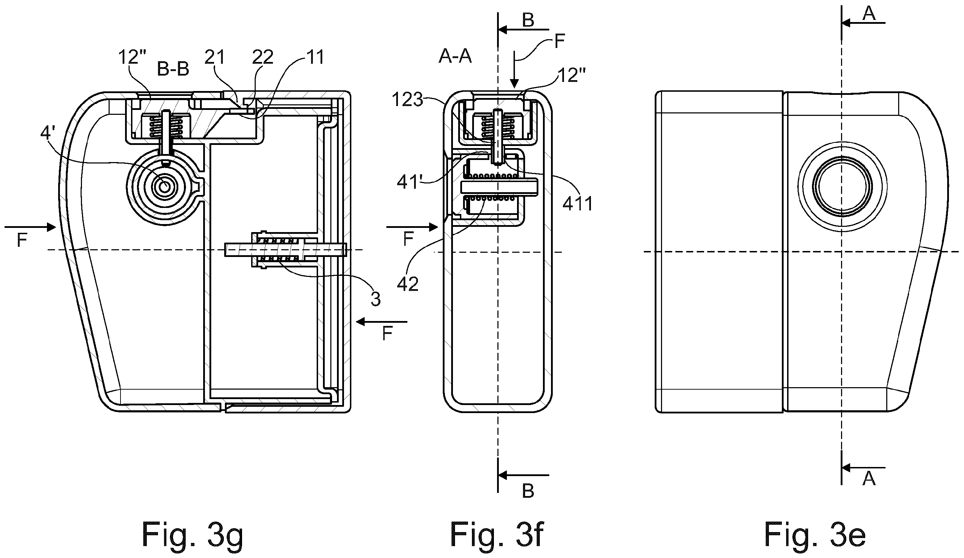

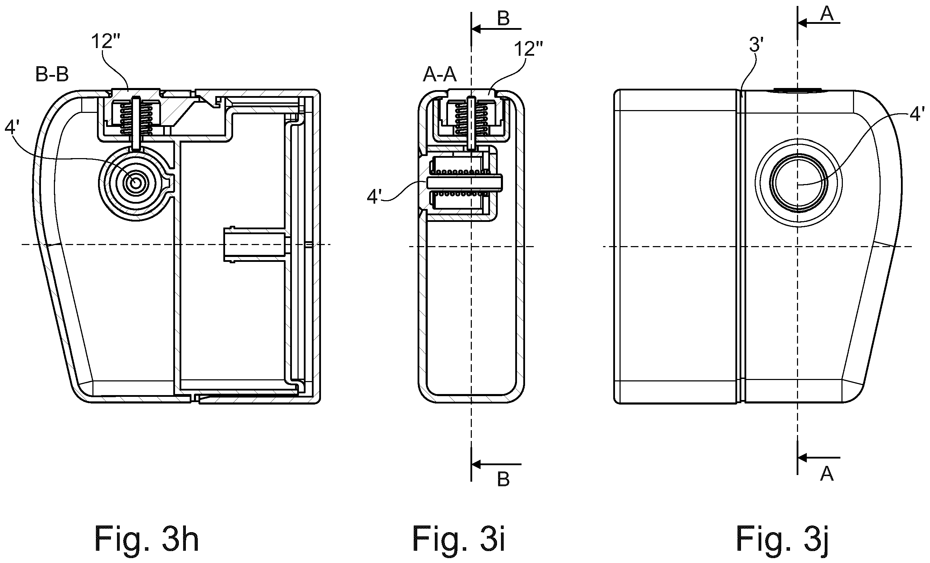

Der Sicherheitsmechanismus der Figuren 3 zum lösbaren Verriegeln der Akkueinheit 1 mit der Kontrolleinheit 2 umfasst einen Rasthaken 21, eine Rastöffnung 11 und ein Betätigungselement 12". Das Betätigungselement 12" sowie die Rastöffnung 11 sind Teil der Akkueinheit 1. Der Rasthaken 21 ist Teil der Kontrolleinheit 2. Figuren 3b bis 3d zeigen das VAD-System in einer Ruheposition. Figuren 3e bis 3g zeigen das VAD-System in der Freigabeposition.

Der Rasthaken 21 ist integral mit dem Gehäuse der Kontrolleinheit 2 ausgebildet. Der Rasthaken weist eine Rastnase 22 auf. Das Betätigungselement 12" ist integral mit der Rastöffnung 11 ausgebildet. In der in den Figuren 3b bis 3d dargestellten Ruheposition ragt der Rasthaken 21 durch die Rastöffnung 11 hindurch. Die Rastnase 22 hintergreift eine Umrandung der Rastöffnung 11, sodass eine Betätigung des Betätigungselements 12" in einer Richtung II gesperrt ist. Das Betätigungselement 12" weist eine Feder 122 auf, die das Betätigungselement in die in Fign. 3b bis 3d gezeigte Position drängt. Zum Betätigen des Betätigungselements 12" wird die Feder 122 zusammengedrückt, wie in Fig. 3f ersichtlich ist.

Die Akkueinheit 1 weist ein Sicherheitselement 4' auf. Das Sicherheitselement 4' ist von einer Sicherheitsposition in eine Löseposition verbringbar. Das Sicherheitselement 4' sperrt in der Sicherheitsposition eine Betätigung des Betätigungselements 12" und gibt in der Löseposition eine Betätigung des Betätigungselements 12" frei. Das Sicherheitselement 4' ist als Druckknopf ausgebildet. In den Figuren 3b bis 3d ist das Sicherheitselement 4 in der Sicherheitsposition gezeigt. Eine Hülse 41' des Sicherheitselements 4' sperrt die Betätigung des Betätigungselements 12". Das Sicherheitselement 4' weist eine Feder 42 auf, die das Sicherheitselement 4' in die Sicherheitsposition drängt. Zum Verbringen des Sicherheitselements 4' in die Löseposition muss ein Nutzer eine Kraft F auf das Sicherheitselement 4' aufbringen, die die Feder 42 zusammendrückt (wie in Fig. 3f gezeigt). In den Figuren 2e bis 2g ist das Sicherheitselement 4 in der Löseposition gezeigt. Ein Nutzer bringt eine Kraft F auf das Sicherheitselement auf, sodass die Hülse 41' derart verschoben ist, dass eine Öffnung 411 in der Hülse 41' koaxial gegenüber einem Stift 123 des Betätigungselements 12" angeordnet ist. Der Stift 123 des Betätigungselements 12" kann dann in die Öffnung des Sicherheitselements 4' verschoben werden, wenn das Sicherheitselement 4' betätigt ist, sodass das Sicherheitselement 4' eine Betätigung des Betätigungselements 12" freigibt.

Ausgehend von der in Figuren 3b bis 3d gezeigten Ruheposition wurden die Akkueinheit 1 und die Kontrolleinheit 2 entlang der Achse a zusammengedrückt, um die Akkueinheit 1 bzw. die Kontrolleinheit 2 in eine Vorlöseposition zu verbringen. In der Vorlöseposition ragt der Rasthaken 21 weiterhin durch die Rastöffnung 11. Die Rastnase 22 hintergreift jedoch nicht mehr die Umrandung der Rastöffnung 11.

Zum Verbringen der Akkueinheit 1 und Kontrolleinheit in die Freigabeposition (gezeigt in den Figuren 3e bis 3g), müssen die Akkueinheit 1 und die Kontrolleinheit 2 also zunächst zusammengedrückt werden, sodass sie sich in der Vorlöseposition befinden. Daraufhin wird das Sicherheitselement 4' betätigt, sodass die Betätigung des Betätigungselements 12" freigegeben ist. Dann wird das Betätigungselement 12" betätigt, wodurch die Rastöffnung nach unten verschoben wird, sodass sich der Rasthaken 21 außerhalb der Rastöffnung 11 (vgl. Fign. 3e bis 3g) befindet. Nun können die Akkueinheit 1 und die Kontrolleinheit 2 voneinander gelöst werden.

Die Figur 3h zeigt in Schnittansicht BB, die Figur 3i in Schnittansicht AA und Figur 3j in Draufsicht eine Kontrolleinheit 2 und eine Akkueinheit 1, die denen der Figuren 3a-g entspricht, wobei anstelle einer Feder 3 ein elastisches Element 3' in Form eines elastischen Dichtelements, beispielsweise ein Moosgummi-Ring, zwischen der Kontrolleinheit 2 und der Akkueinheit 1 angeordnet ist. Die oben in Bezug auf die vorherigen Figuren beschriebenen weiteren Merkmale treffen daher auch auf das in Figuren 3h-j gezeigte VAD-System zu. Dabei zeigen Fig. 3h-j die Kontrolleinheit 2 und die Akkueinheit 1 in einem zusammengesetzten und verriegelten Zustand in der Ruheposition.

Merkmale, die in Bezug auf eines der Ausführungsbeispiele beschrieben wurden, können in anderen Ausführungsbeispielen zusätzlich oder alternativ zu anderen Merkmalen realisiert sein. Die oben beschriebenen Ausführungsbeispiele sind nicht einschränkend. Für einen Fachmann ergibt sich, dass die beschriebenen Merkmale anderweitig miteinander kombiniert werden können.

## Patentansprüche

1. Sicherheitsmechanismus zum Verriegeln und Lösen einer ersten mit einer zweiten Einheit, insbesondere zum Verriegeln und Lösen einer Ackueinheit (1) eines VAD-Systems mit einer Kontrolleinheit (2) eines VAD-Systems, der Sicherheitsmechanismus umfassend einen Rasthaken (21) aufweisend eine Rastnase (22), eine Rastöffnung (11) und ein erstes Betätigungselement (12)
wobei
- in einer Ruheposition der Rasthaken (21) in die Rastöffnung (11) derart eingreift, dass der Rasthaken (21) durch die Rastöffnung (11) hindurchragt und die Rastnase (22) eine Umrandung der Rastöffnung (11) zumindest teilweise hintergreift,
- in einer Vorlöseposition der Rasthaken (21) zumindest teilweise in die Rastöffnung (11) derart eingreift, dass zumindest ein Teil des Rasthakens (21) durch die Rastöffnung hindurchragt und die Rastnase (22) von der Umrandung der Rastöffnung (11) gelöst ist,
- in einer Freigabeposition der Rasthaken (21) außerhalb der Rastöffnung (11) angeordnet ist,
wobei das Betätigungselement derart ausgebildet ist, dass eine Betätigung des Betätigungselements (12) den Rasthaken (21) und/oder die Rastöffnung (11) von der Vorlöseposition in die Freigabeposition bewegt.

2. Sicherheitsmechanismus gemäß Anspruch 1, **dadurch gekennzeichnet, dass** der Rasthaken (21) und die Rastöffnung (11) derart ausgebildet sind, dass in der Ruheposition der Rasthaken (21) mit der Rastöffnung (11) in einer ersten Richtung und in einer zweiten Richtung, die insbesondere perpendikulär zur ersten Richtung ist, verriegelt ist und der Rasthaken (21) in eine Richtung zum Verbringen in die Vorlöseposition bewegbar ist.

3. Sicherheitsmechanismus gemäß Anspruch 2, **dadurch gekennzeichnet, dass** der Rasthaken (21) in der Vorlöseposition in die Rastöffnung (11) derart eingreift, dass der Rasthaken (21) mit der Rastöffnung (11) in der ersten Richtung verriegelt ist und in der zweiten Richtung gelöst ist.

4. Sicherheitsmechanismus gemäß einem der vorhergehenden Ansprüche, **dadurch gekennzeichnet, dass** eine Betätigung des Betätigungselements (12) durch die Rastnase (22) in der Ruheposition gesperrt ist.

5. Sicherheitsmechanismus gemäß einem der vorhergehenden Ansprüche, **dadurch gekennzeichnet, dass** das Betätigungselement (12) als Druckelement derart ausgebildet ist, dass eine auf das Betätigungselement ausgeübte Druckkraft den Rasthaken (21) und/oder die Rastöffnung (11) derart bewegt, dass der Rasthaken (21) von der Vorlöseposition in die Freigabeposition verbracht wird.

6. Sicherheitsmechanismus gemäß einem der vorhergehenden Ansprüche, **dadurch gekennzeichnet, dass** das Betätigungselement integral mit dem Rasthaken (21) oder integral mit der Rastöffnung (11) ausgebildet ist.

7. Sicherheitsmechanismus gemäß einem der vorhergehenden Ansprüche, **gekennzeichnet durch** ein elastisches Element (3), das derart ausgebildet und angeordnet ist, dass es den Rasthaken (21) von der Vorlöseposition in die Ruheposition drängt.

8. Sicherheitsmechanismus gemäß einem der vorhergehenden Ansprüche, **gekennzeichnet durch** einen zweiten Rasthaken (21'), eine zweite Rastöffnung (11') und ein zweites Betätigungselement (12'), die insbesondere spiegelsymmetrisch zum ersten Rasthaken (21), zur ersten Rastöffnung (11) und zum ersten Betätigungselement (12) angeordnet sind.

9. Sicherheitsmechanismus gemäß Anspruch 8, **dadurch gekennzeichnet, dass** die Betätigungselemente (12, 12') gegenüber angeordnet sind, insbesondere derart, dass ein gleichzeitiges Zusammendrücken der Betätigungselemente (12, 12') bewirkt, dass sich die Rasthaken (21, 21') in der Freigabeposition befinden.

10. Sicherheitsmechanismus gemäß einem der vorherigen Ansprüche, **gekennzeichnet durch** ein Sicherheitselement (4), das von einer Sicherheitsposition in eine Löseposition verbringbar ist, wobei das Sicherheitselement (4) in der Sicherheitsposition eine Betätigung des Betätigungselements (12, 12', 12") sperrt und in der Löseposition eine Betätigung des Betätigungselements (12, 12', 12") freigibt.

11. Sicherheitssystem umfassend eine erste Einheit und eine zweite Einheit, wobei die erste Einheit mit der zweiten Einheit über einen Sicherheitsmechanismus gemäß einem der vorherigen Ansprüche lösbar verbindbar ist, wobei der Rasthaken (21) fest mit der ersten Einheit verbunden ist und die Rastöffnung (11) fest mit der zweiten Einheit verbunden ist

12. Sicherheitssystem gemäß Anspruch 11, **dadurch gekennzeichnet, dass** die erste Einheit und die zweite Einheit in der Ruheposition und in der Vorlöseposition über den Sicherheitsmechanismus miteinander verriegelt sind und in der Freigabeposition voneinander gelöst sind.

13. VAD-System, umfassend ein Sicherheitssystem gemäß einem der Ansprüche 11 bis 12, wobei die erste Einheit eine Kontrolleinheit (2) ist und die zweite Einheit eine Akkueinheit (1) ist oder umgekehrt.

14. Verfahren zum Lösen einer ersten Einheit von einer zweiten Einheit eines Sicherheitssystems gemäß einem Ansprüche 11 oder 12 oder eines VAD-Systems gemäß Anspruch 13, umfassend die Schritte
I. Bewegen der ersten Einheit in Richtung der zweiten Einheit,
II. Betätigen des Betätigungselements (12, 12', 12"),
III. Lösen der ersten Einheit von der zweiten Einheit,
wobei Schritt II auf Schritt I folgt und Schritt III auf Schritt II folgt, um die erste Einheit von der zweiten Einheit zu lösen.

15. Verfahren gemäß Anspruch 14, wobei zwischen den Schritten I und II ein Sicherheitselement (4, 4') von einer Sicherheitsposition in eine Löseposition verbracht wird, wobei das Sicherheitselement (4, 4') in der Sicherheitsposition eine Betätigung des Betätigungselements (12, 12', 12") sperrt und in der Löseposition eine Betätigung des Betätigungselements (12, 12', 12") freigibt.
